# FASCICULE DE BREVET EUROPEEN

(11) **EP 4 297 652 B1**
(45) Date de publication et mention de la délivrance du brevet: **10.12.2025**
(21) Numéro de dépôt: 22704546.5
(22) Date de dépôt: 17.02.2022
(51) Int. Cl.: A61B 5/245, A61B 5/00, G01R 33/26, G01N 24/00

(54) **SYSTÈME DE POSITIONNEMENT ET DE MAINTIEN D'UN CAPTEUR DE RÉFÉRENCE AUTOUR D'UN CASQUE DE MAGNÉTOENCÉPHALOGRAPHIE**
SYSTEM ZUR POSITIONIERUNG UND AUFRECHTERHALTUNG DER POSITION EINES REFERENZSENSORS UM EINEN MAGNETENZEPHALOGRAFIEHELM
SYSTEM FOR POSITIONING AND MAINTAINING THE POSITION OF A REFERENCE SENSOR AROUND A MAGNETOENCEPHALOGRAPHY HELMET

(30) Priorité: 25.02.2021 FR 2101839
(43) Date de publication de la demande: 03.01.2024
(73) Titulaire: Commissariat à l'Energie Atomique et aux Energies Alternatives, 75015 Paris (FR)
(72) Inventeur: LABYT, Etienne, 38054 GRENOBLE Cedex 09 (FR); FOURCAULT, William, 38054 GRENOBLE Cedex 09 (FR); PAQUIN-HONORE, Ilea, 59000 LILLE (FR); LAFFONT, Guilhem, 59000 LILLE (FR)
(74) Mandataire: Ipsilon
(86) Numéro de dépôt international: PCT/EP2022/053901
(87) Numéro de publication internationale: WO 2022/179925

(56) Documents cités:
- CN-A- 110 742 607
- CN-U- 210 203 501
- US-A1- 2020 315 482
- US-A1- 2021 011 094

## Description

### Domaine technique

La présente invention concerne le domaine général de la magnétoencéphalographie (MEG).

Elle a trait plus particulièrement à la fixation d'un capteur de référence sur un casque MEG.

Bien que décrite en référence à une application où le dispositif MEG est un casque, le dispositif MEG peut être une ceinture thoracique de magnétocardiographie ou une ceinture abdomino-pelvienne de magnétoencéphalographie ou de magnétocardiographie fœtale.

### Technique antérieure

Les magnétomètres à pompage optique (OPM) commencent à être utilisés dans des prototypes de magnétoencéphalographie qui sont des dispositifs d'enregistrement du champ magnétique cérébral : [1], [2], [3].

Ces capteurs sont physiquement indépendants les uns des autres et peuvent être positionnés au plus près du cuir chevelu du patient. L'intérêt est d'optimiser le ratio signal à bruit puisque le champ magnétique décroit avec la distance. Cela permet aussi de conserver une position relative des capteurs par rapport à la tête qui est fixe.

Un autre moyen d'améliorer la qualité de signal mesuré est de disposer d'un capteur de référence placé à distance des capteurs de mesure du signal cérébral : [4].

Dans les casques MEG à base de capteurs SQUID (acronyme anglais pour *« Superconducting QUantum Interference Device* »), cette approche a déjà été utilisée.

Mais, du fait de la configuration des équipements, conditionnée par l'utilisation de fluide cryogénique (hélium liquide), la matrice de capteurs SQUID mesurant le signal cérébral était située au sein d'un casque rigide et des capteurs de référence étaient placés, par exemple, en haut du vase d'hélium liquide. On pourra se référer aux équipements MEG commercialisés sous la dénomination commerciale CTF.

Les distances et positions relatives du ou des capteurs de référence par rapport aux capteurs de mesures SQUID étaient donc définies d'avance et définitivement figées.

Or, dans le cas d'un casque MEG à capteurs OPM pour la mesure, ces derniers sont portés directement sur la tête du patient. L'utilisation d'un capteur de référence nécessite donc de pouvoir le positionner à distance des capteurs OPM de mesure tout en assurant son maintien de façon fixe par rapport à ceux-ci, quels que soient les mouvements de la tête du patient. Le brevet JP2020151023 décrit et revendique un support monté sur la tête, une fixation du capteur et un capteur de type OPM placé en regard de la région du cortex définie par l'aire de Broadman.

Le brevet CN105147289 décrit et revendique lui un casque MEG en matière élastique fixé à la tête par une mentonnière. La demande de brevet US2021/011094A1 décrit un dispositif d'étalonnage détachable sur un casque MEG. La demande de brevet US2020/0315482A1 décrit un dispositif de mesure magnétique et un dispositif de mesure magnétique monté sur la tête.

La demande de brevet WO2020/084194A1 revendique un système de casque rigide, adaptable à différentes tailles de tête et permettant de positionner des OPM en régions temporales droite et gauche, au-dessus des oreilles de l'utilisateur, pour l'enregistrement de réponses cérébrales à un stimulus auditif.

Il existe un besoin pour améliorer les systèmes de fixation des capteurs de référence sur les casques MEG, notamment ceux des systèmes existants, tels que mentionnés ci-avant, lorsque les capteurs de mesure sont des capteurs OPM.

Le but général de l'invention est alors de répondre au moins en partie à ce besoin.

### Exposé de l'invention

Pour ce faire, l'invention a tout d'abord pour objet, un système de positionnement et de maintien d'un capteur de référence sur un casque de magnétoencéphalographie MEG, comprenant :
- un arceau comprenant :
   au moins une branche de fixation pour fixer l'arceau à un casque MEG,
   une platine-support sur laquelle est fixée la branche,
- un poteau-support du capteur fixé à la platine-support de l'arceau;
- une pièce de verrouillage pour fixer le capteur de référence au poteau selon au moins une position définissant la position par rapport à un casque MEG.

Selon un mode de réalisation avantageux, l'arceau comprend deux branches agencées symétriquement l'une de l'autre par rapport à la platine-support.

De préférence, le poteau est un poteau creux s'étendant selon un axe longitudinal (X) dans lequel le capteur de référence peut être inséré selon plusieurs positions longitudinales.

Selon une variante de réalisation, le creux du poteau comprend une pluralité de fentes de calage, espacées longitudinalement, formant chacune un support d'une cale de positionnement du capteur selon une position longitudinale.

La pièce de verrouillage est de préférence une bague de verrouillage à serrer autour du poteau creux.

Selon une variante de réalisation avantageuse, l'arceau comprend des goulottes de positionnement de câbles de capteurs OPM fixés sur le casque MEG.

Chaque branche de fixation comprend de préférence à son extrémité libre une platine à visser sur le casque MEG au moyen d'une molette de serrage.

Avantageusement, l'arceau, le poteau et la pièce de verrouillage sont en matière plastique, de préférence en polyamide.

L'invention concerne enfin un ensemble comprenant un casque de magnétoencéphalographie MEG auquel est fixé le système de positionnement et de maintien décrit précédemment.

Selon une variante avantageuse, le casque comprend au moins un point d'ancrage sur lequel est fixée la branche de fixation au moyen d'une molette de serrage, le point d'ancrage étant un pan incliné muni d'un trou percé et taraudé dans laquelle la mollette de serrage est vissée.

Ainsi, l'invention consiste essentiellement en un système de positionnement et de maintien d'un capteur de référence, permettant de positionner ce capteur de référence à différentes distances d'un casque de magnétoencéphalographie supportant des capteurs OPM de mesure du signal cérébral.

Ce système comprend essentiellement un arceau destiné à se fixer en au moins un point, voir en deux points sur le casque MEG et d'un poteau se fixant, de préférence au sommet, du casque souple dans laquelle on vient placer le capteur de référence.

Ce poteau intègre de préférence des fentes de calage définissant plusieurs positions prédéfinies pour permettre de positionner le capteur de référence à différentes distances des capteurs OPM mesurant le signal cérébral.

La pièce de verrouillage permet de maintenir la position du capteur de référence.

De préférence, la fixation du système selon l'invention est faite directement sur le casque souple portant les capteurs OPM de mesure permettant ainsi au capteur de référence de garder une distance et une position relative fixe par rapport aux capteurs OPM de mesure, quels que soient les mouvements de tête du patient.

D'autres avantages et caractéristiques ressortiront mieux à la lecture de la description détaillée, faite à titre illustratif et non limitatif, en référence aux figures suivantes.

### Brève description des dessins

[Fig 1] la figure 1 est une vue en perspective d'un arceau d'un système de positionnement selon l'invention.
[Fig 2] la figure 2 est une autre vue en perspective de l'arceau selon la figure 1.
[Fig 3] la figure 3 est une vue d'une molette de fixation du système à un casque MEG selon l'invention.
[Fig 4] la figure 4 est une vue en perspective montrant une pièce de fixation d'un casque MEG pour la fixation de l'arceau.
[Fig 5] la figure 5 est une vue en perspective d'un poteau du système de positionnement selon l'invention.
[Fig 6] la figure 6 est une vue en perspective d'une bague de verrouillage du capteur de référence.
[Fig 7] la figure 7 est une vue en perspective d'une pièce de calage en positionnement longitudinal du capteur de référence dans le poteau.
[Fig 8] la figure 8 est une vue en perspective du système de positionnement selon l'invention fixé au casque MEG.
[Fig 9] la figure 9 est une autre vue en perspective du système de positionnement selon l'invention fixé au casque MEG, avec un capteur de référence positionné et fixé.
[Fig 10] la figure 10 est une vue en perspective du système de positionnement selon l'invention avec un capteur de référence fixé au moyen d'une bague de serrage sur le poteau-support.

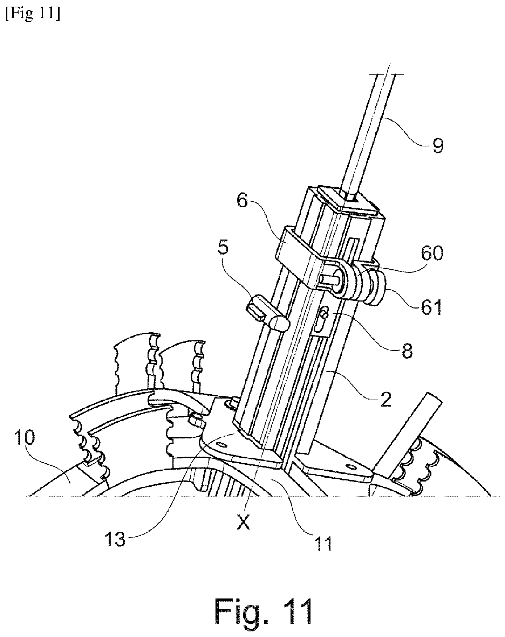

[Fig 11] la figure 11 reprend la figure 10 avec en outre la présence d'une pièce de calage en hauteur du capteur de référence dans le poteau.

### Description détaillée

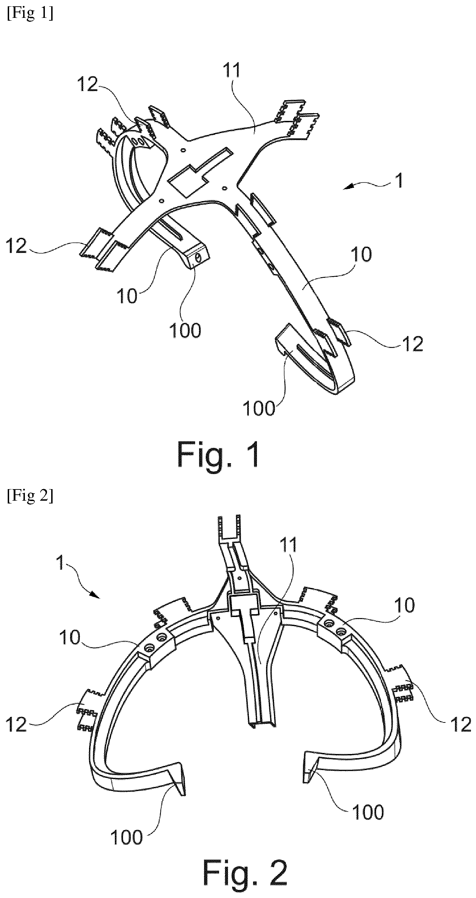

Les figures 1 et 2 montrent un arceau 10 du système de positionnement et de maintien du capteur de référence selon l'invention.

L'arceau 10 est conçu de façon à être suffisamment souple pour pouvoir s'adapter aux différentes morphologies de tête : en fonction de la morphologie, l'arceau doit pouvoir plus ou moins s'arquer et suffisamment rigide pour permettre un bon maintien d'un poteau-support 2 du capteur comme détaillé par la suite.

L'arceau 10 peut être réalisé en matière plastique, typiquement en PLA.

Cet arceau 10 comprend deux branches de fixation 10 agencées symétriquement de part et d'autre une platine support 11.

Chaque branche 10 est de préférence une branche agencée latéralement par rapport à la platine-support 11.

Chaque branche 10 présente une extrémité libre 100 percée pour la fixation à un point d'ancrage 3.

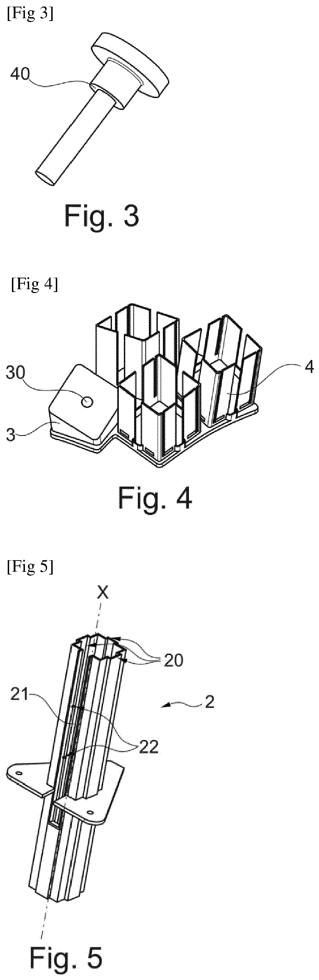

Chaque extrémité libre 100 peut venir se fixer au moyen d'une molette de serrage 40 montrée en figure 3 sur un point d'ancrage prévu au niveau d'un casque MEG.

La figure 4 montre un exemple de point d'ancrage 3 tel qu'il peut être agencé dans un casque MEG.

Le point d'ancrage 3 est un pan incliné muni d'un trou 30 percé et taraudé en son centre dans laquelle la mollette de serrage est vissée. Un point d'ancrage 3 est de préférence agencé dans le casque MEG, de part et d'autre de la tête, en arrière des oreilles et en regard des zones du crâne appelées mastoïdes.

L'inclinaison du pan 3 est conçue pour s'adapter à la zone de fixation située à chaque extrémité 100 des branches latérales 10 de l'arceau.

Comme illustré en figure 4, chaque point d'ancrage 3 fait partie d'un bloc d'un nombre de trois plots support 4 de capteurs de mesure OPM du casque MEG.

L'arceau 10 comprend également des goulottes 12 sur chacune de ses branches 10 et de préférence sur la platine-support 11. Ces goulottes 12 permettent de positionner les câbles des capteurs OPM de mesure situés sur la tête du patient, afin de les maintenir stables et d'éviter tout effet de traction sur les capteurs de mesure OPM. Ceci permet de garantir un niveau de bruit de mesure faible.

La figure 5 montre un poteau creux 2 pour le support du capteur de référence.

Ce poteau 2 est fixé à la platine-support 11 de l'arceau 10 par l'intermédiaire d'une plaque de fixation 13.

Ce poteau 2 est à section carrée. Il est ajouré sur une face et les trois autres faces sont rainurées en leur centre, afin d'éloigner la matière de certains éléments sensibles du capteur de référence.

Le poteau 2 comprend une partie évidée centrale 21. Cette partie évidée ne s'étend de préférence que sur une partie substantielle de sa hauteur, c'est-à-dire pas jusqu'en bas du poteau 2 de façon à avoir une fixation stable rigide sur un emplacement de sonde prévu à cet effet un casque MEG. Cette partie évidée 21 permet d'insérer le capteur de référence. Des fentes calage 22 sont agencées le long du poteau à différentes positions longitudinales. Ces fentes 22 permettent de positionner longitudinalement le capteur de référence à plusieurs distances différentes des capteurs OPM de mesure placés sur le scalp du patient.

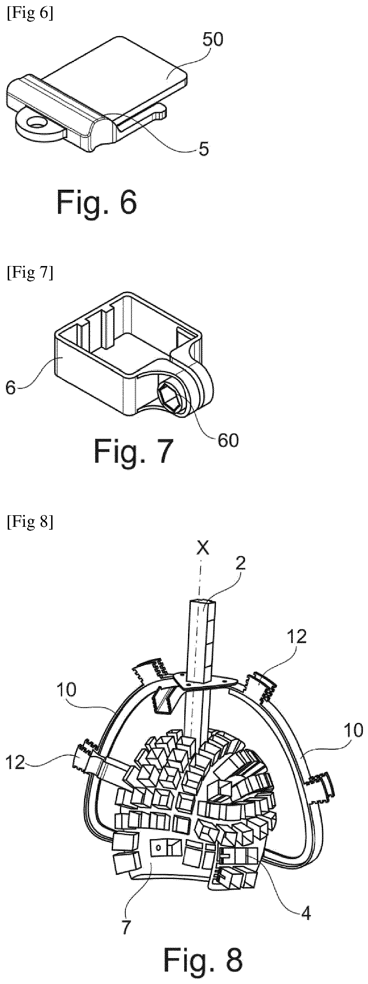

Une pièce de calage 5 montrée en figure 6 permet de régler la hauteur choisie en s'insérant dans le poteau 2 au niveau de l'une ou l'autre des fentes 22 pour une position donnée.

Une bague de verrouillage 6 montrée en figure 7 vient se fixer autour du poteau 2 pour verrouiller et maintenir le capteur de référence en position fixe par rapport aux capteurs OPM de mesure et ce, quels que soient les mouvements de tête du patient.

Plus précisément, la pièce de calage 5 est constituée d'une languette 50 qui vient s'insérer dans une des deux fentes 22 du poteau 2 à la position longitudinale choisie.

Un capteur de référence est glissé à l'intérieur du poteau 2 et repose alors sur cette pièce de cale 5.

La bague de verrouillage 6 est ensuite positionnée en regard de la partie haute du capteur de référence, autour du poteau 2 et elle est serrée à l'aide d'une vis.

Le système de positionnement selon l'invention qui vient d'être décrit est fixé à un casque MEG 7 pouvant accueillir un capteur OPM de mesure du signal cérébral, comme suit.

La base du poteau 2 est fixée sur un plot support, de type de celui référencé à la figure 4, au sommet du casque MEG.

L'arceau 1 est fixé en deux points d'ancrage latéraux sur le casque MEG.

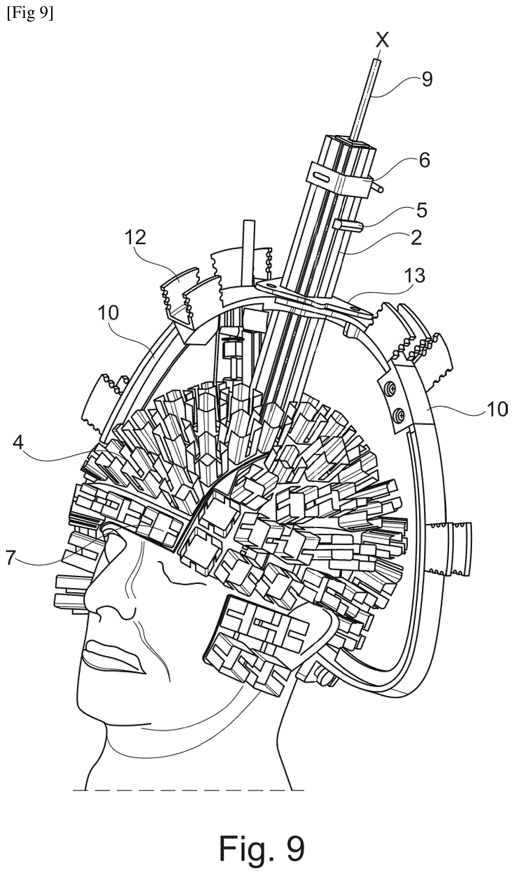

Les figures 8 et 9 montrent un casque MEG avec ses plots-supports 4 de capteurs OPM et le système de positionnement et de maintien d'un capteur de référence 8 qui y est fixé.

Dans une réalisation concrète, le système de positionnement et de maintien du capteur de référence décrit a été conçu pour s'adapter sur un casque souple MEG 7 comprenant un nombre de 97 plots-support 4 pour les capteurs OPM de mesure du signal cérébral.

Sur la figure 9, un capteur de référence 8 relié à son câble 9 est inséré dans le poteau 2 et y est verrouillé et maintenu en position fixe au moyen de la bague 6 serrée au moyen d'une molette de serrage 61 vissée dans les œillets de serrage 60.

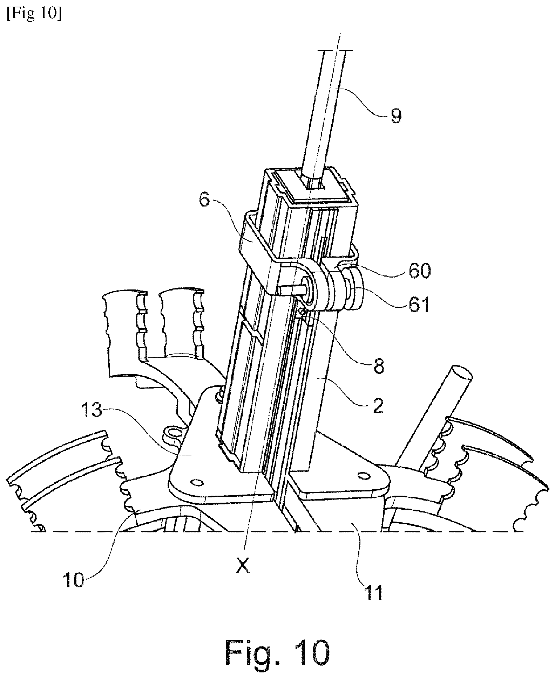

La figure 10 reprend la figure 9 en montrant en outre la pièce de calage 5 qui insérée dans les fentes de calage 22 supporte et maintient le capteur de référence 8, inséré dans le poteau 2, dans une position longitudinale donnée.

L'invention n'est pas limitée aux exemples qui viennent d'être décrits; on peut notamment combiner entre elles des caractéristiques des exemples illustrés au sein de variantes non illustrées.

D'autres variantes et améliorations peuvent être envisagées sans pour autant sortir du cadre de l'invention.

Dans l'exemple illustré de la figure 8, le poteau-support 2 est fixé à la platine-support 11 de l'arceau 1 de sorte à ce qu'il soit sensiblement positionné au sommet de la tête d'une personne sur laquelle la magnétoencéphalographie doit être réalisée. Bien entendu, on peut envisager d'autres positions de fixation autour du crâne d'une personne.

Dans l'exemple illustré de la figure 4, les points d'ancrage 3 sont prévus au niveau des mastoïdes. Dans des déclinaisons de MEG fœtale et magnétocardiographie (MCG), on peut envisager de positionner ces points d'ancrage 3 respectivement au niveau des crêtes iliaques (MEG fœtale) ou des acromions (MCG).

### Liste des références citées:

[1]: Boto et al., « Moving magnetoencephalography towards real-world applications with a wearable system ». Nature. 2018 Mar 29;555(7698):657-661 DOI: 10.1038/nature26147.
[2]: Hill et al., « Multi-Channel Whole-Head OPM-MEG »: Helmet design and a comparison with a conventional system NeuroImage Vol 219, 1 Oct 2020, 116995https://doi.org/10.1016/j.neuroimage.2020.116995.
[3]: Borna et al, «Non-Invasive Functional-Brain-Imaging with an OPM-based Magnetoencephalography System» PLoS ONE 15(1). 2020 https://doi.org/10.1371/journal.pone.0227684.
[4]: A.A. FifeJ. Vrba, S.E. Robinson[...] W. Sutherling. "Synthetic gradiometer systems for MEG." Jul 1999 IEEE Transactions on Applied Superconductivity.

## Revendications

1. Système de positionnement et de maintien d'un capteur de référence (8) sur un casque (7) de magnétoencéphalographie (MEG), comprenant :
- un arceau (1) comprenant :
au moins une branche de fixation (10) pour fixer l'arceau à un casque MEG,
une platine-support (11) sur laquelle est fixée la branche,
- un poteau-support (2) du capteur fixé à la platine-support de l'arceau ;
- une pièce de verrouillage (6) pour fixer le capteur de référence au poteau selon au moins une position définissant la position par rapport à un casque MEG.

2. Système (1) selon la revendication 1, l'arceau comprenant deux branches (10) agencées symétriquement l'une de l'autre par rapport à la platine-support.

3. Système (1) selon la revendication 1 ou 2, le poteau (2) étant un poteau creux s'étendant selon un axe longitudinal (X) dans lequel le capteur de référence peut être inséré selon plusieurs positions longitudinales.

4. Système (1) selon la revendication 3, le creux (21) du poteau comprenant une pluralité de fentes de calage (22), espacées longitudinalement, formant chacune un support d'une cale de positionnement (5) du capteur selon une position longitudinale.

5. Système (1) selon l'une des revendications 3 ou 4, la pièce de verrouillage étant une bague de verrouillage (6) à serrer autour du poteau creux.

6. Système (1) selon l'une des revendications précédentes, l'arceau comprenant des goulottes (12) de positionnement de câbles de capteurs OPM fixés sur le casque MEG.

7. Système (1) selon l'une des revendications précédentes, chaque branche de fixation comprenant à son extrémité libre (100) une platine à visser sur le casque MEG au moyen d'une molette de serrage (40).

8. Système (1) selon l'une des revendications précédentes, l'arceau, le poteau et la pièce de verrouillage étant en matière plastique, de préférence en polyamide.

9. Ensemble (6) comprenant un casque de magnétoencéphalographie (MEG) auquel est fixé le système de positionnement et de maintien selon l'une des revendications 1 à 8.

10. Ensemble (6) selon la revendication 9, le casque comprenant au moins un point d'ancrage (3) sur lequel est fixée la branche de fixation (10) au moyen d'une molette de serrage (40), le point d'ancrage (3) étant un pan incliné muni d'un trou (30) percé et taraudé dans laquelle la mollette de serrage est vissée.

## Patentansprüche

1. System zur Positionierung und Aufrechterhaltung der Position eines Referenzsensors (8) an einem Magnetenzephalographie(MEG)-Helm (7), beinhaltend:
- einen Bügel (1), beinhaltend:
mindestens einen Befestigungsarm (10) zur Befestigung des Bügels an einem MEG-Helm,
eine Stützplatte (11), an der der Arm befestigt ist,
- einen Stützpfosten (2) für den Sensor, der an der Stützplatte des Bügels befestigt ist;
- ein Verriegelungselement (6), um den Referenzsensor gemäß mindestens einer Position, die die Position in Bezug auf einen MEG-Helm definiert, an dem Pfosten zu befestigen.

2. System (1) nach Anspruch 1, wobei der Bügel zwei Arme (10) beinhaltet, die in Bezug auf die Stützplatte symmetrisch zueinander eingerichtet sind.

3. System (1) nach Anspruch 1 oder 2, wobei der Pfosten (2) ein hohler Pfosten ist, der sich entlang einer Längsachse (X) erstreckt und in den der Referenzsensor gemäß mehreren Längspositionen eingeführt werden kann.

4. System (1) nach Anspruch 3, wobei die Aushöhlung (21) des Pfostens eine Vielzahl von in Längsrichtung beabstandeten Verkeilungsschlitzen (22) beinhaltet, die jeweils eine Stütze für einen Positionierungskeil (5) des Sensors gemäß einer Längsposition bilden.

5. System (1) nach einem der Ansprüche 3 oder 4, wobei das Verriegelungselement ein Verriegelungsring (6) ist, der um den hohlen Pfosten geklemmt wird.

6. System (1) nach einem der vorhergehenden Ansprüche, wobei der Bügel Positionierungsführungen (12) für Kabel von OPM-Sensoren, die an dem MEG-Helm befestigt sind, beinhaltet.

7. System (1) nach einem der vorhergehenden Ansprüche, wobei jeder Befestigungsarm an seinem freien Ende (100) eine Platte beinhaltet, die mit Hilfe eines Klemmrädchens (40) an den MEG-Helm angeschraubt wird.

8. System (1) nach einem der vorhergehenden Ansprüche, wobei der Bügel, der Pfosten und das Verriegelungselement aus Kunststoff, vorzugsweise aus Polyamid, sind.

9. Anordnung (6), die einen Magnetenzephalographie(MEG)-Helm beinhaltet, an dem das System zur Positionierung und Aufrechterhaltung der Position nach einem der Ansprüche 1 bis 8 befestigt ist.

10. Anordnung (6) nach Anspruch 9, wobei der Helm mindestens einen Verankerungspunkt (3) beinhaltet, an dem der Befestigungsarm (10) mit Hilfe eines Klemmrädchens (40) befestigt ist, wobei der Verankerungspunkt (3) eine geneigte Ebene ist, die über ein Gewindebohrloch (30) verfügt, in das das Klemmrädchen eingeschraubt wird.

## Claims

1. System for positioning and maintaining the position of a reference sensor (8) on a magnetoencephalography (MEG) helmet (7), comprising:
- an arch (1) comprising:
at least one fixing branch (10) for fixing the arch to an MEG helmet,
a support plate (11) to which the branch is fixed,
- a sensor support post (2) fixed to the support plate of the arch;
- a locking piece (6) for fixing the reference sensor to the post in at least one position defining the position with respect to an MEG helmet.

2. System (1) according to Claim 1, the arch comprising two branches (10) arranged symmetrically to one another with respect to the support plate.

3. System (1) according to Claim 1 or 2, the post (2) being a hollow post extending on a longitudinal axis (X) in which the reference sensor can be inserted according to several longitudinal positions.

4. System (1) according to Claim 3, the hollow (21) of the post comprising a plurality of wedging slits (22), spaced apart longitudinally, each forming a support for a positioning wedge (5) for positioning the sensor according to a longitudinal position.

5. System (1) according to either of Claims 3 and 4, the locking piece being a locking ring (6) to be tightened around the hollow post.

6. System (1) according to one of the preceding claims, the arch comprising chutes (12) for positioning cables of OPM sensors fixed to the MEG helmet.

7. System (1) according to one of the preceding claims, each fixing branch comprising, at its free end (100), a plate to be screwed onto the MEG helmet by means of a tightening thumbwheel (40).

8. System (1) according to one of the preceding claims, the arch, the post and the locking piece being made of plastic material, preferably of polyamide.

9. Assembly (6) comprising a magnetoencephalography (MEG) helmet to which is fixed the system for positioning and maintaining the position according to one of Claims 1 to 8.

10. Assembly (6) according to Claim 9, the helmet comprising at least one anchor point (3) to which the fixing branch (10) is fixed by means of a tightening thumbwheel (40), the anchor point (3) being an inclined flat provided with a drilled and tapped hole (30) into which the tightening thumbwheel is screwed.
